# EUROPEAN PATENT SPECIFICATION

(11) **EP 2 148 690 B1**
(45) Date of publication and mention of the grant of the patent: **05.08.2015**
(21) Application number: 08753723.9
(22) Date of filing: 23.04.2008
(51) Int. Cl.: A61K 36/889, A61K 31/704, A61P 35/00

(54) **COMPOUNDS EXTRACTED FROM PALM OIL MILL EFFLUENT FOR THE TREATMENT OF CANCER, COMPOSITIONS THEREOF AND METHODS THEREWITH**
AUS DEM ABFLUSS EINER PALMÖLMÜHLE EXTRAHIERTE VERBINDUNGEN ZUR BEHANDLUNG VON KREBS, ZUSAMMENSETZUNGEN DARAUS UND VERFAHREN DAMIT
COMPOSÉS EXTRAITS D'UN EFFLUENT D'INSTALLATION DE TRITURATION D'HUILE DE PALME UTILES POUR LE TRAITEMENT DU CANCER, COMPOSITIONS À BASE DE CES COMPOSÉS ET PROCÉDÉS CORRESPONDANTS

(30) Priority: 23.04.2007 MY 0700623
(43) Date of publication of application: 03.02.2010
(73) Proprietor: Malaysian Palm Oil Board, 43000 Kajang, Selangor (MY)
(72) Inventor: SUNDRAM, Kalyana a/I P. Manickam, Selangor Darul Ehsan (MY); RAVIGADEVI a/p Sambathamurthi, Selangor Darul Ehsan (MY); TAN, Yew, Ai, Selangor Darul Ehsan (MY); BIN WAHID, Mohd, Basri, Selangor Darul Ehsan (MY); LEOW, Soon, Sen, Selangor (MY)
(74) Representative: Lloyd, John Scott
(86) International application number: PCT/MY2008/000034
(87) International publication number: WO 2008/130216

(56) References cited:
- WO-A1-2008/127086
- US-A1- 2004 023 894
- GUTHRIE ET AL: "Abstract 1907: combined effect of palm oil tocotrienols, flavonoids and tamoxifen on the prolferation of estrogen receptor-positive MCF-7 human breast cancer cells" PROCEEDINGS OF THE 87TH. ANNUAL MEETING OF THE AMERICAN ASSOCIATION FOR CANCER RESEARCH. WASHINGTON, APR. 20 - 24, 1996; [PROCEEDINGS OF THE ANNUAL MEETING OF THE AMERICAN ASSOCIATION FOR CANCER RESEARCH], PHILADELPHIA, AACR, US, vol. VOL. 37, no. 37, 1 March 1996 (1996-03-01), page 280, XP002099426
- BIRT D F ET AL: "Dietary agents in cancer prevention: Flavonoids and isoflavonoids" PHARMACOLOGY AND THERAPEUTICS, ELSEVIER, GB LNKD- DOI:10.1016/S0163-7258(01)00137-1, vol. 90, no. 2-3, 1 May 2001 (2001-05-01), pages 157-177, XP002398513 ISSN: 0163-7258
- SRIVASTAVA J.K. AND GUPTA S.: 'Tocotrienol-rich fraction of plam oil induces cell cycle arrest and apoptosis selectively in human prostate cancer cells' BIOCHEMICAL AND BIOPHYSICAL RESEARCH COMMUNICATIONS vol. 346, no. 2, 2006, pages 447 - 453, XP005502292
- NESARENTNAM K. ET AL.: 'Tocotrienol-rich fraction palm oil and gene expression in human breast cancer cells' ANNALS OF THE NEW YORK ACADEMY OF SCIENCES vol. 1031, 2004, pages 143 - 157, XP008125304
- NESARETNAM K. ET AL.: 'Tocotrienol-rich fraction from palm oil affects gene expression in tumors resulting from MCF-7 cell inoculation in athymic mice' LIPIDS vol. 39, no. 5, 2004, pages 459 - 467, XP008126208
- WATTANAPENPAIBOON N. AND WAHLQVIST M.: 'Phytonutrient deficiency: the place of palm fruit' ASIA PACIFIC JOURNAL OF CLINICAL NUTRITION vol. 12, no. 3, 2003, pages 363 - 368, XP009131470
- SUNDRAM K. ET AL.: 'Palm fruit chemistry and nutrition' ASIA PACIFIC JOURNAL OF CLINICAL NUTRITION vol. 12, no. 3, 2003, pages 355 - 362, XP009131159
- KAUSAR H. ET AL.: 'Palm oil alleviates 12-O-tetradecanoyl-phorbol-13-acetate-induc ed tumor promotion response in murine skin' CANCER LETTERS vol. 192, no. 2, 2003, pages 151 - 160, XP008125306
- SYLVESTER P.W. ET AL.: 'Role of GTP-binding proteins in reversing the antiproliferative effects of tocotrienols in preneoplastic mammary epithelial cells' ASIA PACIFIC JOURNAL OF CLINICAL NUTRITION vol. 11, no. SUPPL., 2002, pages S452 - S459, XP008125309
- MCINTYRE B.S. ET AL.: 'Antiproliferative and apoptotic effects of tocopherols and tocotrienols on preneoplastic and neoplastic mouse mammary epithelial cell' PROCEEDINGS OF THE SOCIETY FOR EXPERIMENTAL BIOLOGY AND MEDICINE vol. 224, no. 4, 2000, pages 292 - 301, XP008004370
- NESARETNAM K. ET AL.: 'Effect of tocotrienols on the growth of a human breast cancer cell line in culture' LIPIDS vol. 30, no. 12, 1995, pages 1139 - 1143, XP009131155
- SUNDRAM K. ET AL.: 'Effect of dietary palm oils on mammary carconogenesis in female rats induced by 7,12-dimethylbenz(a)anthracene' CANCER RESEARCH vol. 49, no. 6, 1989, pages 1447 - 1451, XP008125308

## Description

### Field of Invention

The disclosure involves compositions and methods for prevention and treatment of cancer with phenolics including but not confined to phenolic acids and flavonoids derived from plant material. More particularly, the present invention relates to compositions comprising compounds extracted from palm oil mill effluent.

### Background of the Invention

In the prior art, treatment of cancer involves resorting to chemotherapy in certain cases which is usually effective in killing or damaging malignant cells.

At present, there are various methods of being employed to fight cancer, most of which involve conventional therapy or radiation therapy or surgery. However, such treatments harm normal cells also.

Recognizing one of the major drawbacks as mentioned in the earlier paragraph, scientists and inventors of the related industry have been developing less damaging techniques or methods for the prevention and thus treatment of cancer, including utilizing plant-derived compounds which may also impart positive pharmacological by way of their abilities to serve as antioxidants. Generally, antioxidants have various beneficial medical uses, including for fighting cancer cells. Phenolics are powerful antioxidants which provide an effective defense mechanism against free radicals. Vitamin E functions as an antioxidant and protects cells by destroying or "neutralizing" the potentially damaging oxygen molecules called free radicals which are by-products of normal activity of cells.

Crude palm oil contains components including carotenoids, tocopherols, tocotrienols, sterols, triterpene alcohols, phospholipids, glycolipids and terpenic and paraffinic hydrocarbons. Tocopherols and tocotrienols, nutritionally significant components of Vitamin E, play a major role in providing protection for cell components from oxidative damage.

An exemplary of such composition is as disclosed in US20040023894 - Treatment of Inflammatory, Cancer, and Thrombosis Disorders (EL-Naggar et al.) which suggests compositions constituting *Tocotrienol,* an antioxidant of the vitamin E family, rich fraction of Palm oil and citrus for treatment of cancer. In this document, the invention relates to a composition for the treatment of inflammatory, cancer and thrombotic disorders, particularly, in combination, of a COX-2 inhibitor, low dose aspirin and antioxidant , or isoflavones wherein such combination provides anti-cancer efficacy. Nevertheless, this invention works favorably with the combination essentially comprising COX-2 inhibitor and low dose aspirin, in which the aspirin may act as an anti-inflammatory agent. The present invention is distinctive to that described in US20040023894, as it provides a method for prevention and treatment of cancer, which involves using an effective dose of a combination of phenolics including phenolic acids and flavonoids extracted from Palm oil mill effluent, with or without the addition of vitamin E, accompanied with or without conventional chemotherapy or radiation therapy or surgery. The content of phenolic-flavonoid-rich antioxidant complex plays a major role in providing potential benefits against cancer.

It is therefore the object of the present invention to provide a composition comprising phenolics including phenolic acids and flavonoids extracted from palm oil effluent either on their own or in combination with each other or in combination with vitamin E for the prevention and treatment of cancer cells.

It is another object of the present invention to provide a method for administering the composition described herein to a mammal in need thereof.

Further, it is an object of the present invention to provide a natural source composition which is cost effective and uncomplicated to prepare for administration to a mammal in need thereof.

### Summary of the Invention

The invention provides a composition comprising phenolics including phenolic acids and flavonoids obtained from palm oil mill effluents for use in the treatment of cancer by preventing or inhibiting the proliferation of cancer cells, wherein the cancer is colon cancer or prostate cancer.

Disclosed herein is a composition comprising phenolics with or without the addition of vitamin E, in the preparation of a medicament for preventing or inhibiting the growth of a cancer, wherein said phenolics are obtained from palm oil mill effluent.

Disclosed herein is the use of therapeutically effective amount of a composition as described herein, with or without vitamin E, in the preparation of a medicament for preventing or inhibiting the growth of cancer in an individual by administering to an individual in need thereof.

### Detailed Description of the Present Invention

Disclosed herein is a composition comprising phenolics including phenolic acids and flavonoids and the usage of the said composition in a medicament for preventing and treating cancer.

The term "phenolics" refers to a class of compounds produced by plants grouped together due to their chemical structure.

Accordingly, the method disclosed herein involves administering an effective dose of phenolics with or without Vitamin E to an individual identified as being at enhanced risk for cancer and/or having cancer, in order to prevent and/or to treat cancer. Phenolics including phenolic acids and flavonoids remain in the palm oil mill effluent resulting from the heat inactivation of polyphenol oxidase enzyme during the milling process. Vitamin E may comprise tocotrienols and tocopherols, wherein the tocotrienols or tocopherols can be alpha, beta, gamma or delta- isomers.

The following specific examples will provide detailed illustrations of methods of producing the drugs and the related pharmaceutical dosage units.

Administration of palm phenolics protects against skin melanoma. Possible administrations include oral ingestion of the palm phenolics, pharmaceutical dose forms or through their topical applications.

The extract of substantially pure phenolics can be delivered in any form appropriate for oral ingestion, pharmaceutical dose forms or topical application. Such forms include solutions, colloidal dispersions, oil-in-water or water-in-oil suspensions, creams, gels, lotions, powders, etc. The methodology for formulation of different vehicle types is well known in the art and can be found in any relevant standard literature.

### Palm phenolics protect against/human breast cancer.

Human breast cancer cells were cultured *in vitro* either in the presence or absence of various doses (wt %) of palm phenolics. The palm phenolics inhibited the growth of human breast cancer cells in a dose dependent manner whereas breast cancer cells that were not supplemented with palm phenolics continued proliferation.

### Palm phenolics protect against human prostate cancer.

Human prostate cancer cells were cultured in vitro either in the presence or absence of various doses (wt %) of palm phenolics. The palm phenolics inhibited the growth of human prostate cancer cells in a dose-dependent manner whereas prostate cancer cells that were not supplemented with palm phenolics continued to proliferate.

### Palm phenolics protect against colon cancer

Using an animal model (rat) and Dimethyl hydrazine (DMH) as the chemical trigger, colon carcinogenesis was induced in the rats through multiple doses of DMH intra-gastrically. The experimental group of animals were supplemented with palm phenolics as part of their drinking water whereas the control group of animals was provided water and not supplemented with any palm phenolics. All rats were maintained ad libitum on normal rat chow for the duration of the trial. Rats supplemented with the palm phenolics demonstrated significantly lower colon polyps and tumors compared to the control animals not supplemented with these palm phenolics.

### Microarray Studies on Tumour-Bearing Mice Supplemented with Oil Palm Phenolics Suggest a Cytostatic Effect

Male inbred *balb*/*c* mice at around 6 weeks of age were each injected subcutaneously at the dorsum of the neck with 200 µL of J558 myeloma cells (2-3 x 10⁶ cells/200 µL) after acclimatization for 1 week. The mice were then divided into two groups, one given distilled water (*n = 6*) and the other given oil palm phenolics (1500 ppm gallic acid equivalent) (*n = 6*). The mice were sacrificed after 4 weeks, when the presence of tumours was observed through palpation at the neck. Tumours were excised, snap-frozen in liquid nitrogen and stored at - 80°C until the total RNA isolation process. After determination of yield, purity and integrity of the RNA isolated, 4 total RNA samples with the highest integrity within each condition were then selected for microarray studies using the Illumina MouseRef-8 Expression BeadChip (Illumina, Inc., San Diego, CA), as per manufacturer's instructions.

Quality control of the hybridization, microarray data extraction and initial analysis were carried out using the Illumina BeadStudio software (Illumina, Inc., San Diego, CA). Outlier samples were removed via hierarchical clustering analysis provided by the Illumina BeadStudio software and also using the TIGR MeV software [1], via different distance metrics. A minimum of 3 replicates per condition (with outliers removed) was then considered for further analysis. Gene expression values were normalized using the rank invariant method and genes which had a Detection Level of more than 0.99 in either the control or treatment samples were considered significantly detected. To filter the data for genes which changed significantly in terms of statistics, the Illumina Custom error model was used and genes were considered significantly changed at a |Differential Score| of more than 13, which was equivalent to a P Value of less than 0.05.

The genes and their corresponding data were then exported into the Microsoft Excel software for further analysis. To calculate fold changes, an arbitrary value of 10 was given to expression values which were less than 10. Fold changes were then calculated by dividing means of Signal Y (treatment) with means of Signal X (control) if the genes were up-regulated and vice versa if the genes were down-regulated. Two-way (gene and sample) hierarchical clustering of the significant genes was then performed using the TIGR MeV [1] software to ensure that the replicates of each condition were clustered to each other. The Euclidean distance metric and average linkage method were used to carry out the hierarchical clustering analysis.

Changes in biological pathways and gene ontologies were assessed via functional analysis, using the GenMAPP [2] and MAPPFinder [3] softwares. The MAPPFinder software ranks GenMAPPs (pathways) and gene ontologies based on the hypergeometric distribution. Readers are referred to [3] for further explanations of the terms used in the MAPPFinder software. GenMAPPs and gene ontologies which had Permuted P Values of less than 0.05, Numbers of Genes Changed of more than or equal to 2 and Z Scores of more than 2 were considered significant. Boxes coloured yellow indicate genes which were up-regulated while those coloured blue indicate genes which were down-regulated. Individual boxes which have different shadings within them indicate the presence of multiple probes (splice transcripts) within a single gene.

Using a P Value of less than 0.05, we found that 815 genes were differentially expressed in tumours of mice supplemented with oil palm phenolics, with 391 up-regulated and 424 down-regulated. Functional analysis of these genes indicated that those involved in the cell cycle (Figure 1) were differentially expressed. Genes such as *Ccnd2, Ccne2, Cdk4* and *Cdk2* were up-regulated while *Ccna2, Ccnb1, Plkl, Mad221, Cdc20, Hdac6* and *Mcm 6* were down-regulated. The regulation of these genes thus suggests that oil palm phenolics may inhibit tumour growth by inducing a G1/S phase arrest in the cell cycle

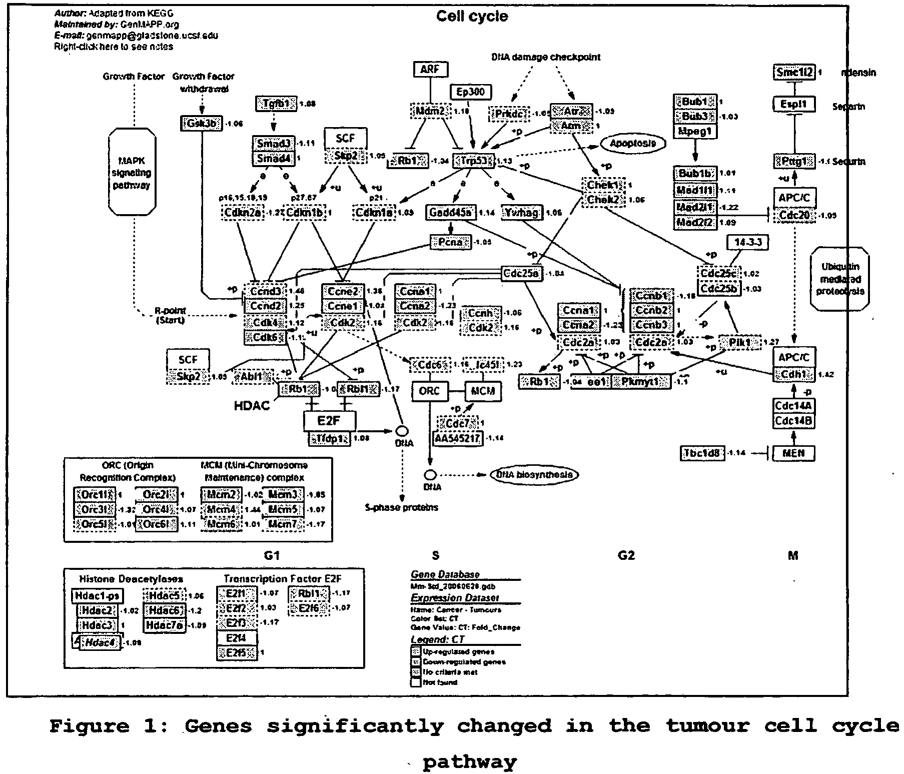

### DOSAGE AND FORMULATION

In addition to direct use of an extract, it is also possible to use different fractions of the oil palm phenolic compounds. What constitutes an effective amount of an extract, or an active portion thereof, will depend on the purity of the extract. For example, if a crude phenolic-containing extract of about 10% purity is employed, the extract will normally be used at a higher concentration. At a higher level of purity, a smaller percentage will be required to achieve the same effect

In accordance to the present invention, the phenolics or Vitamin E may be formulated into pharmaceutical and / or nutraceutical compositions for administration to mammals. Also, they may be provided as compounds with pharmaceutically acceptable carriers. The therapeutic compounds of pharmaceutical and/or nutraceutical compositions may be administered orally or it may be introduced as food supplement. In this context, the amount is preferably selected so as to produce the most effective result devoid of disrupting the original taste of food.

Formulations which are suitable for oral administration include liquid solutions of the active compound dissolved in suitable solvent such as saline, water; capsules or tablets, each containing a predetermined amount of the active agent as solid granules or gelatin; suspensions in an appropriate medium and emulsions.

Patient dosages for oral administration of the phenolics range commonly from 15 mg/day to 1000 mg/day, and typically 200 mg/day. Stated in terms of patient body weight, usual dosages for an average 60kg body weight human, this averages as 3.4 mg/kg/day.

Patient dosages for oral administration of flavonoids range commonly from 15 mg/day to 1000 mg/day and typically 200 mg/day. Stated in terms of patient body weight, usual dosages for an average 60kg body weight human this averages as 3.4-6.7 mg/kg/day.

Patient dosages for oral administration of tocotrienols range commonly from 20 mg/day to 1000 mg/day and typically 200-400 mg/day. Stated in terms of patient body weight, usual dosages for an average 60kg body weight human, this averages as 3.4-6.7 mg/kg/day.

The compound of this invention may be prepared independently, in dosage form as described above, and can also be prepared combined together as combination product.

The term "pharmaceutically and/or nutraceutically acceptable dosage form" as used herein above includes any suitable vehicle for the administration of medications known in the pharmaceutical and/or nutraceutical art, including, by way of example, capsules, tablets, syrups, elixirs and solutions for parenteral injection with specified ranges of drug concentrations.

As used herein, an "effective amount" includes that amount of pharmaceutically acceptable dose sufficient to destroy, modify, control or remove a primary, regional or metastatic cancer cell or tissue; delay or minimize the spread of cancer; or provide a therapeutic benefit in the treatment or management of cancer. These examples are not intended, however, to limit or restrict the scope of the invention in any way and should not be construed as providing conditions, parameters, reagents or starting materials which must be utilized exclusively in order to practice the present invention.

## Claims

1. A composition comprising phenolics including phenolic acids and flavonoids obtained from palm oil mill effluents for use in the treatment of cancer by preventing or inhibiting the proliferation of cancer cells, wherein the cancer is colon cancer or prostate cancer.

2. The composition for use according to Claim 1, wherein the composition further comprises vitamin E.

3. The composition for use according to claim 1, wherein the composition further comprises a pharmaceutically acceptable carrier.

4. The composition for use according to claim 1, wherein the said treatment of cancer further comprises conventional chemotherapy or radiation therapy or surgery.

5. The composition for use according to claim 1, wherein said treatment comprises administering said composition orally or as a food supplement.

6. The composition for use according to claim 1, wherein the cancer is colon cancer.

7. The composition for use according to claim 1, wherein the cancer is prostate cancer.

## Patentansprüche

1. Zusammensetzung, umfassend Phenole, einschließlich Phenolsäuren und Flavonoide, erhalten aus Palmölmühlenabwasser, zur Verwendung in der Behandlung von Krebs durch Verhinderung oder Hemmung der Proliferation von Krebszellen, wobei der Krebs Darmkrebs oder Prostatakrebs ist.

2. Zusammensetzung zur Verwendung nach Anspruch 1, wobei die Zusammensetzung ferner Vitamin E umfasst.

3. Zusammensetzung zur Verwendung nach Anspruch 1, wobei die Zusammensetzung ferner einen pharmazeutisch verträglichen Träger umfasst.

4. Zusammensetzung zur Verwendung nach Anspruch 1, wobei die Behandlung von Krebs ferner konventionelle Chemotherapie oder Strahlentherapie oder Operation umfasst.

5. Zusammensetzung zur Verwendung nach Anspruch 1, wobei die Behandlung die Verabreichung der Zusammensetzung oral oder als ein Nahrungsergänzungsmittel umfasst.

6. Zusammensetzung zur Verwendung nach Anspruch 1, wobei der Krebs Darmkrebs ist.

7. Zusammensetzung zur Verwendung nach Anspruch 1, wobei der Krebs Prostatakrebs ist.

## Revendications

1. Composition comprenant des phénoliques incluant des acides phénoliques et des flavonoïdes obtenus à partir d'effluents de moulin à huile de palme à utiliser dans le traitement du cancer en empêchant ou en inhibant la prolifération de cellules cancéreuses, dans laquelle le cancer est le cancer de la prostate ou le cancer du côlon.

2. Composition à utiliser selon la revendication 1, dans laquelle la composition comprend en outre de la vitamine E.

3. Composition à utiliser selon la revendication 1, dans laquelle la composition comprend en outre un vecteur acceptable d'un point de vue pharmaceutique.

4. Composition à utiliser selon la revendication 1, dans laquelle ledit traitement du cancer comprend en outre une chimiothérapie ou une thérapie par rayonnement ou une chirurgie classique.

5. Composition à utiliser selon la revendication 1, dans laquelle ledit traitement comprend l'administration de ladite composition oralement ou en tant que complément alimentaire.

6. Composition à utiliser selon la revendication 1, dans laquelle le cancer est le cancer du côlon.

7. Composition à utiliser selon la revendication 1, dans laquelle le cancer est le cancer de la prostate.
